# EUROPEAN PATENT APPLICATION

(11) **EP 4 657 069 A1**
(43) Date of publication of application: **03.12.2025**
(21) Application number: 25152394.0
(22) Date of filing: 16.01.2025
(51) Int. Cl.: G01N 33/574, G01N 33/68, G01N 33/92

(54) **APPLICATION OF BIOMARKER IN PREPARING METABOLIC DYSFUNCTION-ASSOCIATED STEATOTIC LIVER DISEASE CLASSIFICATION PRODUCTS**

(30) Priority: 28.05.2024 CN 202410674429
(71) Applicant: The First Affiliated Hospital of the Chinese People's Liberation Army Army Medical University, Chongqing 400038 (CN); Chengdu Seventh People's Hospital, Chengdu, Sichuan 610000 (CN)
(72) Inventor: CHAI, Jin, Chongqing, 400038 (CN); JIANG, Zhongyong, Chengdu, 610000 (CN); DING, Jingjing, Chongqing, 400038 (CN); ZHANG, Xiaoxun, Chongqing, 400038 (CN)
(74) Representative: Bayramoglu et al.

(57) **Abstract**

An application of a biomarker in preparing metabolic dysfunction-associated steatotic liver disease classification products is provided, which belongs to the technical field of biomarkers.. The biomarker is any one or more of the following: a combination of liver protein biomarkers, a combination of serum protein biomarkers, a combination of serum lipid biomarkers, a combination of serum metabolite biomarkers, a combination of serum multiomics (including protein, lipid and metabolite) biomarkers, a combination of urine protein biomarkers, a combination of urine metabolite biomarkers, and a combination of urine multiomics (including protein and metabolite) biomarkers; and the metabolic dysfunction-associated steatotic liver disease is divided into a metabolically active type, a high-risk type of cirrhosis and a high-risk type of hepatocellular carcinoma. The combinations of biomarkers provided by the present disclosure have a good effect on the diagnosis of three MASLD molecular subtypes, which provides technical support for the classification and diagnosis of MASLD.

## Description

### TECHNICAL FIELD

The present disclosure relates to the technical field of biomarkers, and in particular to an application of a biomarker in preparing metabolic dysfunction-associated steatotic liver disease classification products.

### BACKGROUND

Metabolic dysfunction-associated fatty liver disease (MASLD) was formerly known as metabolic-associated fatty liver disease (MAFLD) or non-alcohol fatty liver disease (NAFLD). Its prevalence has increased dramatically worldwide, from 25.3% in 1990-2006 to 38.2% in 2016-2019, and it is predicted that the global prevalence will reach 55.7% by 2040. The MASLD disease spectrum includes simple steatosis and metabolic dysfunction associated steatohepatitis (MASH), and MASH was formerly known as non-alcoholic steatohepatitis (NASH). MASH can further develop into liver fibrosis, cirrhosis and hepatocellular carcinoma (HCC).

However, the pathogenesis of MASLD is complex and its understanding is limited. Until recently, the US Food and Drug Administration (FDA) approved Resmetirom as the first drug for treating NASH. However, after treatment with the maximum dose of Resmetirom, only 29.9% of NASH patients were treated. It is unclear which MASLD patients Resmetirom has therapeutic effects on. In addition, many studies have reported that nuclear receptor famesoid X receptor (FXR) agonists (such as obeticholic acid, OCA) and peroxisome proliferator-activated receptor α

(PPARα) agonists (such as fenofibrate) can improve hepatic steatosis and fibrosis in NASH mouse models. However, in most NASH patients, the therapeutic effect of OCA or fenofibrate treatment is poor, which may be due to species differences between humans and rodents, or the high heterogeneity of human MASLD, because the susceptibility of MASLD is highly variable and is affected by a variety of risk factors, including age, genotype, gender, race, diet, etc.

In order to solve the problem of high heterogeneity of MASLD, several MASLD classification models based on clinical or genetic phenotypes have been proposed. For example, (1) MASLD is defined as 'lean MASLD' (Asian BMI < 23 kg/m²) and 'obese MASLD' subtype based on body mass index (BMI). However, long-term cohort studies have shown that both 'obese MASLD' and 'lean MASLD' can progress to NASH, cirrhosis, and eventually HCC. (2) According to whether the patient is accompanied by PNPLA3 1148 M mutation, MASLD is defined as 'PNPLA3 MASLD' subtype. However, patients with 'NPLA3 MASLD' may also carry other gene mutations that promote the progression of NASLD (such as TM6SF2, MBOAT7, etc.). Therefore, it is necessary to establish a new MASLD classification model to address its high heterogeneity and identify biomarkers for MASLD subtypes, thereby providing strategies for diagnosis and treatment.

### SUMMARY

An objective of the present disclosure is to provide an application of a biomarker in preparing metabolic dysfunction-associated steatotic liver disease classification products, to address the issue of high heterogeneity in metabolic dysfunction-associated fatty liver disease (MASLD) and identify biomarkers for MASLD subtypes, providing a foundation for the diagnosis and treatment of MASLD.

In order to achieve the above objective, the present disclosure adopts the following technical solutions.

The present disclosure provides an application of a biomarker in preparing metabolic dysfunction-associated steatotic liver disease classification products, and the biomarker is any one or more of the following: a combination of liver protein biomarkers, a combination of serum protein biomarkers, a combination of serum lipid biomarkers, a combination of serum metabolite biomarkers, a combination of serum multiomics (including protein, lipid and metabolite) biomarker, a combination of urine protein biomarkers, a combination of urine metabolite biomarkers, and a combination of urine multiomics (including protein and metabolite) biomarkers; and
a classification of a metabolic dysfunction-associated steatotic liver disease is divided into a metabolically active type, a high-risk type of cirrhosis and a high-risk type of hepatocellular carcinoma.
Preferably, the products include a kit;
the combination of the liver protein biomarkers is a combination of laminin beta 1 (LAMB 1), filamin C (FLNC) and excision repair cross-complementation group 3 (ERCC3);
the combination of the serum protein biomarkers is a combination of carboxypeptidase M (CPM), nucleobindin 1 (NUCB1), keratin 17 (KRT17) and Galectin-10 (CLC);
the combination of the serum lipid biomarkers is a combination of free fatty acid (FFA) (18:1), FFA (19:0), ceramide (Cer) (t18:0/24:0) and triacylglycerol (TG) (16:0_16:0_18:1);
the combination of the serum metabolite biomarkers is a combination of FFA (11:1), phosphatidylcholine (PC) (18:1 (9Z)/20:4 (5Z, 8Z, 11Z, 14Z)), diglycerides (DG) (18:3 (9Z,12Z,15Z)/22:4 (7Z, 10Z, 13Z, 16Z)/0:0), Carboxyphosphamide and DG (14:1(9Z)/24:1 (15Z)/0:0);
the combination of the serum multiomics (including protein, lipid and metabolite) biomarkers is a combination of 17alpha, 20alpha-Dihydroxycholesterol, DG (20:1(11Z)/18:1(11Z)/0:0), TG (16:0_16:1_18:1), PC (18:1(9Z)/20:4(5Z,8Z,11Z,14Z)) and reticulon 4 receptor-like 2 (RTN4RL2);
the combination of the urine protein biomarkers is a combination of lipopolysaccharide binding protein (LBP), CD300 antigen-like family member A (CD300A) and GTP cyclohydrolase 1 feedback regulator (GCHFR);
the combination of the urine metabolite biomarkers is a combination of 7alpha-Hydroxyandrost-4-ene-3, 17-dione, 27-Hydroxycholesterol, monomethyl phosphatidylethanolamine (PE-NMe) (15:0/15:0), Chenodeoxycholic acid sulfate and phosphatidylethanolamine (PE) (18:1(11Z)/20:0); and
the combination of the urine multiomics (including protein and metabolite) biomarkers is a combination of ICOS ligand (ICOSLG), 7alpha-Hydroxy-5beta-cholstan-3-one, cytochrome b reductase 1 (CYBRD1), Fc gamma receptor III-A (FCGR3A) and aggrecan (ACAN).

The present disclosure provides a combination of liver protein biomarkers for the metabolic dysfunction-associated steatotic liver disease classification, and the combination of the liver protein biomarkers is a combination of LAMB 1, FLNC and ERCC3.

The present disclosure provides a combination of serum protein biomarkers for the metabolic dysfunction-associated steatotic liver disease classification, and the combination of the serum protein biomarkers is a combination of CPM, NUCB1, KRT17 and CLC.

The present disclosure provides a combination of serum lipid biomarkers for the metabolic dysfunction-associated steatotic liver disease classification, and the combination of the serum lipid biomarkers is a combination of FFA (18:1), FFA (19:0), Cer (t18:0/24:0) and TG (16:0_16:0_18:1).

The present disclosure provides a combination of serum metabolite biomarkers for the metabolic dysfunction-associated steatotic liver disease classification, and the combination of the serum metabolite biomarkers is a combination of FFA (11:1), PC (18:1 (9Z)/20:4 (5Z, 8Z, 11Z, 14Z)), DG (18:3 (9Z,12Z,15Z)/22:4 (7Z, 10Z, 13Z, 16Z)/0:0), Carboxyphosphamide and DG (14: 1(9Z)/24:1 (15Z)/0:0).

The present disclosure provides a combination of serum multiomics (including protein, lipid and metabolite) biomarkers for the metabolic dysfunction-associated steatotic liver disease classification, and the combination of the serum multiomics (including protein, lipid and metabolite) biomarkers is a combination of 17alpha, 20alpha-Dihydroxycholesterol, DG (20:1(11Z)/18:1 (11Z)/0:0), TG (16:0_16:1_18:1), PC (18:1(9Z)/20:4 (5Z,8Z,11Z,14Z)) and RTN4RL2.

The present disclosure provides a combination of urine protein biomarkers for the metabolic dysfunction-associated steatotic liver disease classification, and the combination of the urine protein biomarkers is a combination of LBP, CD300A and GCHFR.

The present disclosure provides a combination of urine metabolite biomarkers for the metabolic dysfunction-associated steatotic liver disease classification, and the combination of the urine metabolite biomarkers is a combination of 7alpha-Hydroxyandrost-4-ene-3,17-dione, 27-Hydroxycholesterol, PE-NMe (15:0/15:0), Chenodeoxycholic acid sulfate and PE (18:1 (11Z)/20:0); and

The present disclosure provides a combination of urine multiomics (including protein and metabolite) biomarkers for the metabolic dysfunction-associated steatotic liver disease classification, and the combination of the urine multiomics (including protein and metabolite) biomarkers is a combination of ICOSLG, 7alpha-Hydroxy-5beta-cholstan-3-one, CYBRD1, FCGR3A and ACAN.

The present disclosure provides a more accurate MASLD classification from the molecular level, namely metabolically active type (MASLD-mSI), high-risk type of cirrhosis (MASLD-mSII) and high-risk type of hepatocellular carcinoma (MASLD-mSIII), and the MASLD classification provided by the present disclosure provides a theoretical basis for the personalized treatment of MALSD patients.

At the same time, the present disclosure further provides a variety of biomarker combinations for the MALSD classification, including a combination of liver protein biomarkers, a combination of serum protein biomarkers, a combination of serum lipid biomarkers, a combination of serum metabolite biomarkers, a combination of serum multiomics (including protein, lipid and metabolite) biomarkers, a combination of urine protein biomarkers, a combination of urine metabolite biomarkers, and a combination of urine multiomics (including protein and metabolite) biomarkers. The combinations of biomarkers provided by the present disclosure have a good effect on the diagnosis of three MASLD molecular subtypes, which provides technical support for the classification and diagnosis of MASLD.

### BRIEF DESCRIPTION OF THE DRAWINGS

In order to more clearly illustrate the embodiments of the present disclosure or technical solutions in the related art, the accompanying drawings used in the embodiments or the related art will now be described briefly. It is obvious that the drawings in the following description are only the embodiment of the disclosure, and that those skilled in the art can obtain other drawings from these drawings without any creative efforts.
FIG. 1 shows a line chart of a cophenetic coefficient of non-negative matrix factorization analysis in Embodiment 1;
FIG. 2 shows a consensus matrix heatmap of non-negative matrix factorization analysis in Embodiment 1;
FIG. 3 shows MASLD classification results of 103 patients in Embodiment 1;
FIG. 4 shows an enrichment result of differentially expressed proteins with high expression of MASLD-mSI in the metabolic-related signalling pathways compared with MASLD-mSII and MASLD-mSIII in Embodiment 1;
FIG. 5 shows an enrichment result of differentially expressed proteins with high expression of MASLD-mSII in the metabolic-related signalling pathways compared with MASLD-mSI and MASLD-mSIII in Embodiment 1;
FIG. 6 shows an enrichment result of differentially expressed proteins with high expression of MASLD-mSIII in the metabolic-related signalling pathways compared with MASLD-mSI and MASLD-mSII in Embodiment 1;
FIG. 7 shows detecting results of protein level of three MASLD molecular subtypes HCC-related proteins HNRNPA2B 1, KRAS and TERT in Embodiment 1;
FIGS. 8A-8B show determined results of histological characteristics of patients with three MASLD molecular subtypes in Embodiment 1, wherein FIG. 8A shows results of steatosis score, liver ballooning score, lobular inflammation score, liver fibrosis score and NAS score of patients with three MASLD molecular subtypes, and FIG. 8B shows determined results of Severity of steatosis, severity of liver ballooning, severity of lobular inflammation, severity of liver fibrosis, and severity of NAS of patients with three MASLD molecular subtypes;
FIG. 9 shows results of serum biochemical analysis of patients with three MASLD molecular subtypes in Embodiment 1;
FIG. 10 shows an identification result of an optimal liver protein biomarker combination of three MASLD subtypes in Embodiment 2;
FIG. 11 shows results of fluorescence staining and quantitative analysis of liver paraffin sections of MASLD patients in Embodiment 2;
FIG. 12 shows accuracy results of three MASLD molecular subtypes identified by multiple immunofluorescence of liver sections of LAMB 1, FLNC and ERCC3 in Embodiment 2;
FIG. 13 shows AUC values of the three MASLD molecular subtypes identified by multiple immunofluorescence of liver sections of LAMB 1, FLNC and ERCC3 in Embodiment 2;
FIG. 14 shows a flow chart of an optimal biomarker combination including a combination of serum protein biomarkers, a combination of serum lipid biomarkers, a combination of serum metabolite biomarkers and a combination of serum multiomics (including protein, lipid and metabolite) biomarkers for identifying three MASLD molecular subtypes in Embodiment 2;
FIG. 15 shows an identification result of an optimal combination of serum protein biomarker for three MASLD subtypes in Embodiment 2;
FIG. 16 shows determined results of expression levels of CPM, NUCB1, KRT17 and CLC in serum of patients with three MASLD subtypes in Embodiment 2;
FIG. 17 shows an identification result of an optimal combination of serum lipid biomarkers for three MASLD subtypes in Embodiment 2;
FIG. 18 shows determined results of expression levels of FFA (18:1), FFA (19:0), Cer (t18:0/24:0) and TG (16:0_16:0_18:1) in serum of patients with three MASLD subtypes in Embodiment 2;
FIG. 19 shows an identification result of an optimal combination of serum metabolite biomarkers for three MASLD subtypes in Embodiment 2;
FIG. 20 shows determined results of expression levels of serum FFA (11:1), PC (18:1 (9Z)/20:4 (5Z, 8Z, 11Z, 14Z)), DG (18:3 (9Z, 12Z, 15Z)/22:4 (7Z, 10Z, 13Z, 16Z)/0:0), Carboxyphosphamide and DG (14:1 (9Z)/24:1 (15Z)/0:0) in three MASLD subtypes in Embodiment 2;
FIG. 21 shows identification results of an optimal combination of serum multiomics (including protein, lipid and metabolite) biomarkers for three MASLD subtypes in Embodiment 2;
FIG. 22 shows determined results of expression levels of 17alpha, 20alpha-Dihydroxycholesterol, DG (20:1 (11Z)/18:1 (11Z)/0:0), TG(16:0_16:1_18:1), PC (18:1 (9Z)/20:4 (5Z, 8Z, 11Z, 14Z)) and RTN4RL2 in serum of patients with three MASLD subtypes in Embodiment 2;
FIG. 23 is a flow chart of an optimal biomarker combination including a combination of urine protein biomarkers, a combination of urine metabolome biomarkers, and a combination of urine multiomics (including protein and metabolite) biomarkers for identifying three MASLD molecular subtypes in Embodiment 2;
FIG. 24 shows an identification result of an optimal combination of urine protein biomarkers for three MASLD molecular subtypes in Embodiment 2;
FIG. 25 shows determined results of expression levels of LBP, CD300 A and GCHFR in urine of patients with three MASLD molecular subtypes in Embodiment 2;
FIG. 26 shows an identification result of an optimal combination of urine metabolite biomarkers for three MASLD molecular subtypes in Embodiment 2;
FIG. 27 shows determined results of expression levels of 7alpha-Hydroxyandrost-4-ene-3,17-dione, 27-Hydroxycholesterol, PE-NMe (15:0/15:0), Chenodeoxycholic acid sulfate and PE (18:1 (11Z)/20:0) in urine of patients with three MASLD molecular subtypes in Embodiment 2;
FIG. 28 shows an identification result of an optimal combination of urine multiomics (including protein and metabolite) biomarkers for three MASLD molecular subtypes in Embodiment 2;
FIG. 29 shows determined results of expression levels of ICOSLG, 7alpha-Hydroxy-5beta-cholestane-3-one, CYBRD1, FCGR3A and ACAN in urine of patients with three MASLD molecular subtypes in Embodiment 2;
FIG. 30 shows determined results of expression levels of liver protein LAMB1, FLNC and ERCC3 in patients with three MASLD molecular subtypes in Embodiment 3;
FIG. 31 shows detecting results of histologic characteristics of patients with three MASLD molecular subtypes in Embodiment 3; and
FIG. 32 shows determined results of serum biochemical characteristics of patients with three MASLD molecular subtypes in Embodiment 3.

### DETAILED DESCRIPTION OF THE EMBODIMENTS

In the following, the technical solutions provided by the present disclosure are described in detail in combination with the embodiments, but they cannot be understood as limiting the scope of protection of the present disclosure.

### Embodiment 1

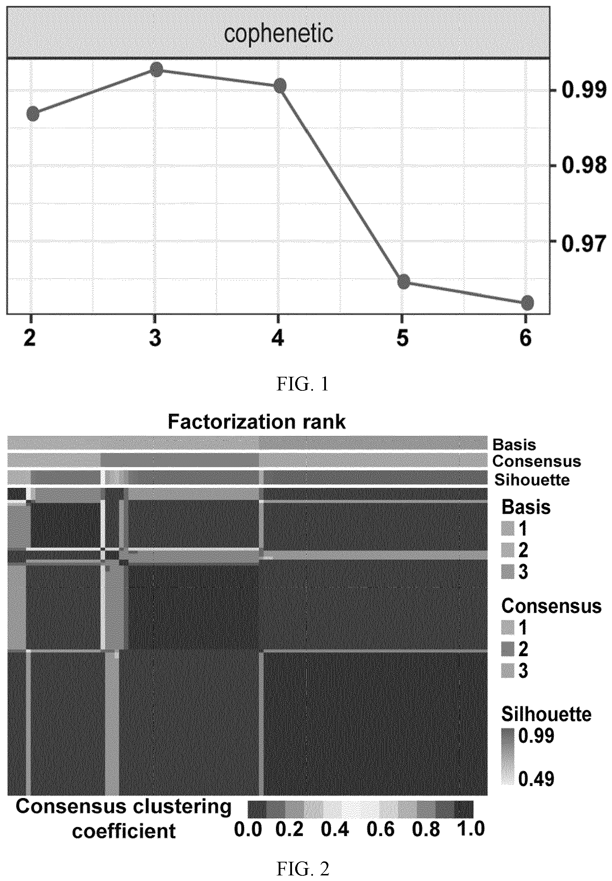

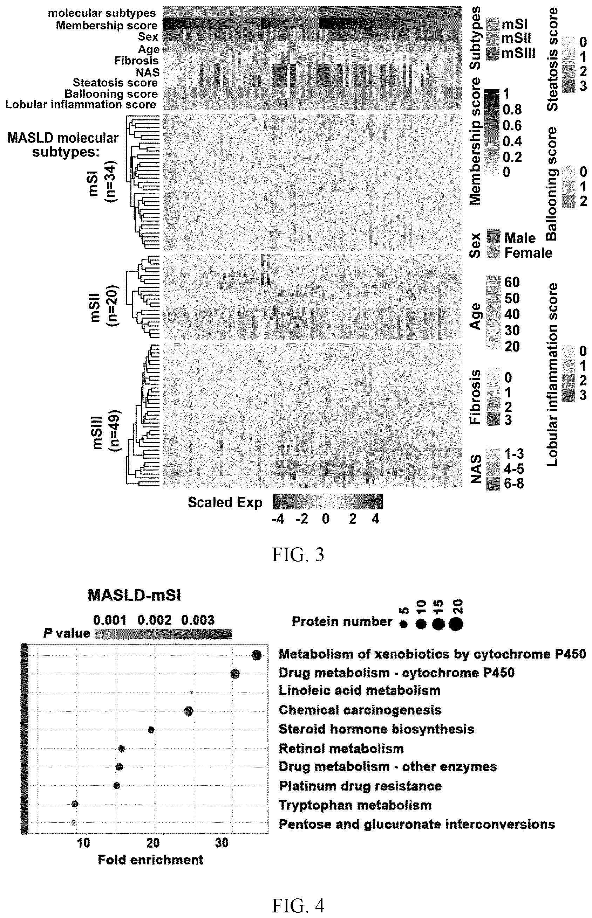

Based on the liver proteome data of 103 MASLD patients, the protein with the highest coefficient of variation of 25% in the liver of MASLD patients was analyzed by non-negative matrix factorization (NMF), and three different MASLD molecular subtypes were analyzed and defined by combining the analysis of a line chart of a cophenetic coefficient and a consensus matrix heatmap, wherein the analysis result of the line chart of the cophenetic coefficient was shown as FIG. 1, and the analysis result of the consensus matrix heatmap was shown as FIG. 2. Three different molecular subtypes of MASLD were named as subtype I (MASLD-mSI), subtype II (MASLD-mSII) and subtype III (MASLD-mSIII), and the classification results of MASLD in 103 patients were shown in FIG. 3.

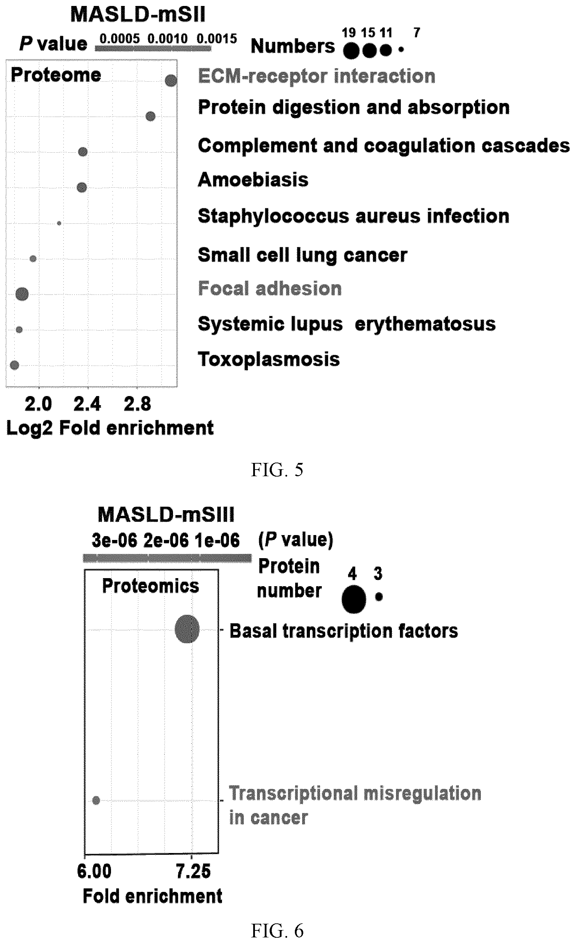

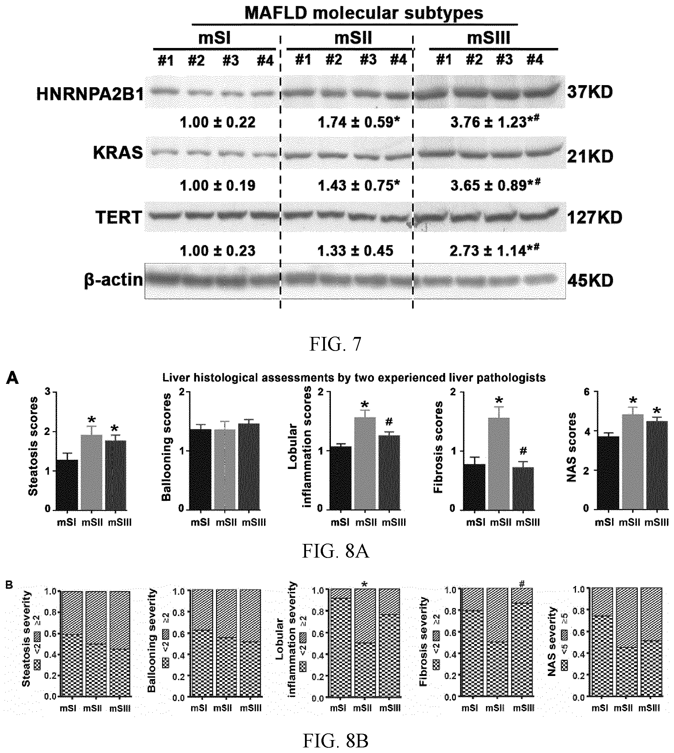

The three MASLD molecular subtypes had unique molecular and histological characteristics. Proteomic analysis of the liver of MASLD patients showed that the differentially expressed proteins with high expression of MASLD-mSI compared with MASLD-mS II and MASLD-mS III were mainly enriched in metabolic-related signalling pathways (FIG. 4), therefore, MASLD-mSI was defined as a metabolically active type; compared with MASLD-mSI and MASLD-mSIII, the differentially expressed proteins with high expression of of MASLD-mSII were significantly enriched in fibrosis-related signalling pathways (FIG. 5), therefore, MASLD-mSII was defined as a high-risk type of cirrhosis; and compared with MASLD-mSI and MASLD-mSII, the differentially expressed proteins with high expression of MASLD-mSIII were mainly enriched in cancer-related signalling pathways (FIG. 6), and the protein levels of HCC-related proteins (HNRNPA2B1, KRAS and TERT) in the MASLD-mSIII were significantly higher than those in the MASLD-mSI and MASLD-mSII (FIG. 7), therefore, MASLD-mSIII was defined as a high-risk type of hepatocellular carcinoma.

The determined results of histologic characteristics of patients with three MASLD molecular subtypes were shown in FIGS. 8A-8B. It could be seen that the steatosis and NAS scores of the MASLD-mSI group were significantly lower than those of the MASLD-mSII and MASLD-mSIII groups, while there was no significant difference in these indexes between the MASLD-mSII and MASLD-mSIII groups, which further reflected the characteristics of active metabolism in the MASLD-mSI. The scores of liver fibrosis and lobular inflammation in the MASLD-mSII group were significantly higher than those in MASLD-mSI and MASLD-mSIII groups, while there was no significant change between MASLD-mSI and MASLD-mSIII groups. The proportion of patients with severe liver fibrosis and inflammation in the MASLD-mSII group was significantly higher than that in the MASLD-mSI and MASLD-mSIII groups, which indicated that the risk of MASLD-mSII progressing to cirrhosis was higher, further illustrating the high risk of cirrhosis of the MASLD-mSII. There was no significant difference in score results for liver ballooning degeneration among the three MASLD molecular subtypes.

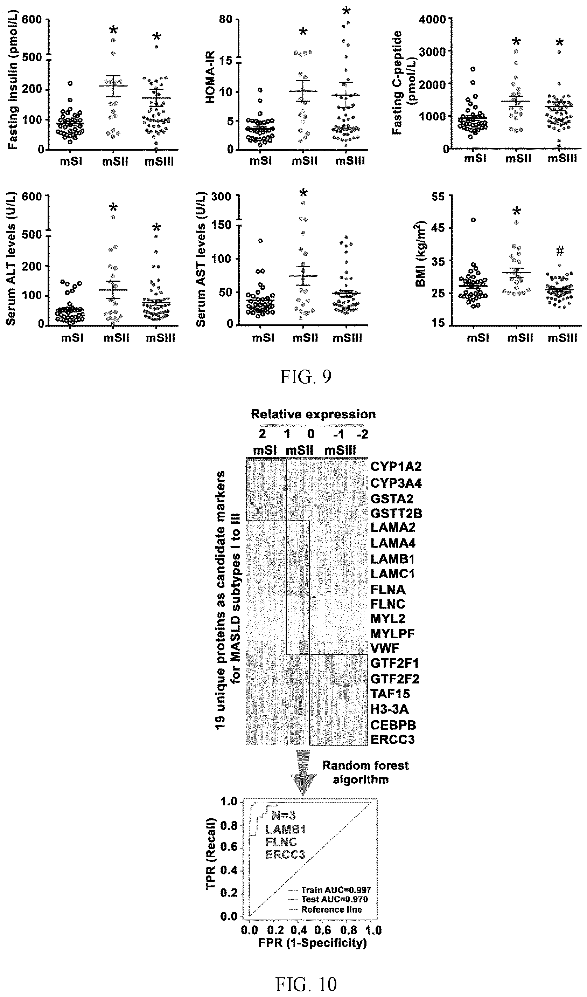

The results of serum biochemical analysis of patients with three MASLD molecular subtypes were shown in FIG. 9. It could be seen that the levels of serum fasting insulin, homeostasis model assessment of insulin resistance (HOMA-IR), fasting plasma c-peptide, alanine aminotransferase (ALT) and aspartate aminotransferase (AST) in the MASLD-mSI group were significantly lower than those in the MASLD-mSII and MASLD-mSIII groups, and there was no significant difference in these indexes between the MASLD-mSII and MASLD-mSIII groups; and the BMI value of MASLD-mSII group was significantly higher than that of the MASLD-mSI and MASLD-mSIII groups, while there was no significant difference in BMI value between the MASLD-mSI and MASLD-mSIII groups.

It could be seen from the above that the three MASLD molecular subtypes proposed in the present disclosure were different from the previous MASLD classification based on clinical phenotype, it was a more in-depth and accurate MASLD classification at the molecular level, which was conducive to providing a basis for personalized treatment of MALSD patients.

### Embodiment 2 Combinations of biomarkers for three molecular subtypes of MASLD

### 1. Combination of liver protein biomarkers

The unique liver proteins (CYP1A2, CYP3A4, GSTA2, GSTT2B, LAMA2, LAMA4, LAMB1, LAMC1, FLNA, FLNC, MYL2, MYLPF, VWF, GTF2F1, GTF2F2, TAF15, H3-3A, CEBPB and ERCC3) of the three MASLD subtypes were used as candidate biomarkers, and an optimal combination of liver protein biomarkers for the three MASLD subtypes was identified by random forest analysis. Model parameters: the RandomForestClassifier function in python language was used, where n estimators = 20, max depth = 2, min samples leaf = 2, and the rest used default values. The output result was the probability that the patient was divided into three MASLD subtypes, and the subtype with the highest probability was the subtype of the patient. The identification result was shown in FIG. 10. It could be seen that the optimal combination of liver protein biomarkers for identifying the three MASLD molecular subtypes was LAMB1, FLNC and ERCC3 (Train AUC = 0.997, and Test AUC = 0.970).

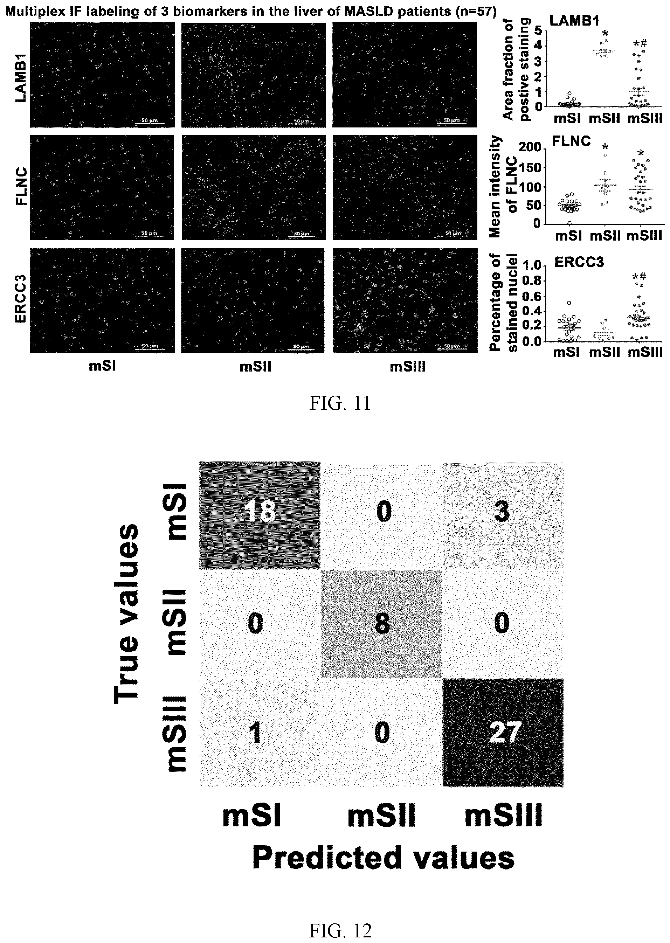

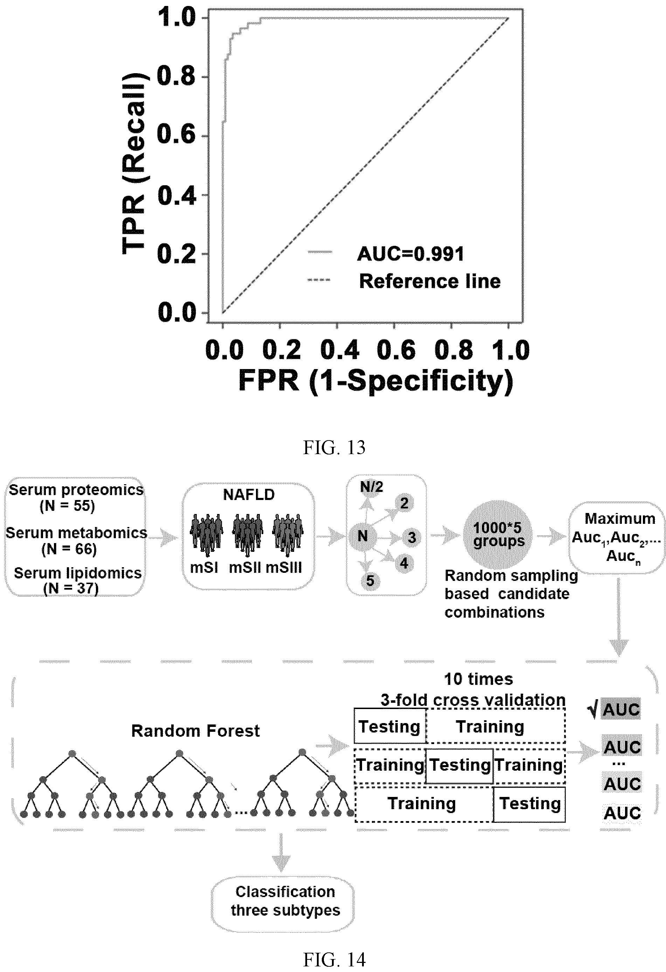

Multiple immunofluorescence staining of LAMB1, FLNC and ERCC3 was performed on liver paraffin sections of 57 patients with MASLD, and quantitative analysis was performed (LAMB1 was used to calculate the positive staining area. FLNC was used to calculate the average optical density, and ERCC3 was used to calculate the percentage of nuclear positive staining), and the results of fluorescent staining and quantitative analysis were shown in FIG. 11. It could be seen from FIG. 11 that the levels of LAMB1 and FLNC in the MASLD-mSII group were significantly higher than those in the MASLD-mSI and MASLD-mSIII groups, and the level of ERCC3 in the MASLD-mSIII group was significantly higher than that in the MASLD-mSI and MASLD-mSII groups. The quantitative values of multiple immunofluorescence staining of LAMB1, FLNC and ERCC3 were analyzed by Random Forest to identify the molecular subtypes of each MASLD patient. Confusion matrix and ROC analysis showed that multiple immunofluorescence of liver sections of LAMB 1, FLNC and ERCC3 could well identify the three molecular subtypes of MASLD. The accuracy was 92.98% (FIG. 12), and the AUC was 0.991 (FIG. 13).

### 2. Combination of serum protein biomarkers, serum lipid biomarkers, serum metabolite and serum multiomics (including protein, lipid and metabolite) biomarkers

Firstly, omics analysis was performed on serum of 78 patients with MASLD on proteins, lipid and metabolism. Characteristic serum proteins, lipids, and metabolites were defined as molecules that were different from the other two molecular subtypes of MASLD.

Afterwards, the characteristic serum proteins, serum lipids, and serum metabolites of the three MASLD molecular subtypes were used as candidate biomarkers, and Random Forest analysis was used. Model parameters: the RandomForestClassifier function in python language was used, where n estimators = 20, max depth = 2, min samples leaf = 2, and the rest used default values. The output result was the probability that the patient belonged to three subtypes of MASLD, respectively, and the subtype with the highest probability was the subtype of the patient. An optimal biomarker combination including a combination of serum protein biomarkers, a combination of serum lipid biomarkers, a combination of serum metabolite biomarkers and a combination of serum multiomics (including protein, lipid and metabolite) biomarkers for identifying three MASLD molecular subtypes was clearly identified. An identification flow chart was shown in FIG. 14.

### 2.1 Combination of serum protein biomarkers

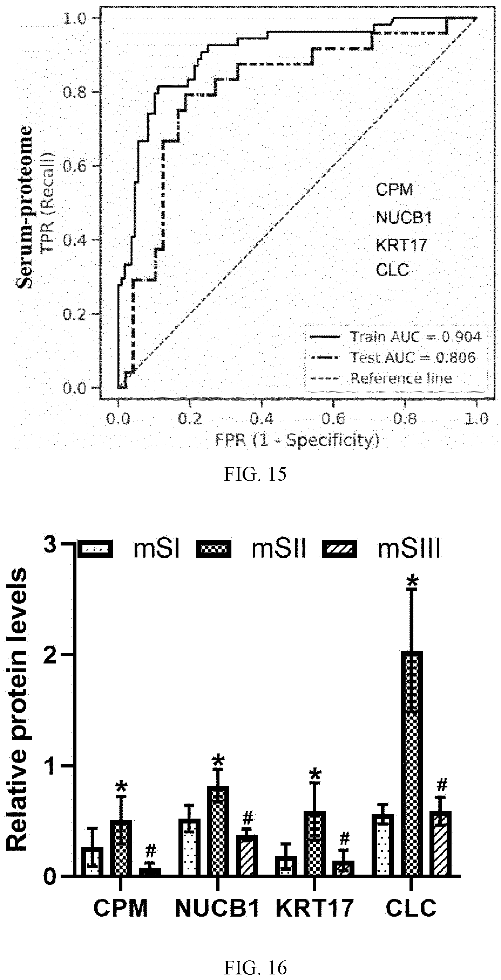

An identification result of an optimal combination of serum protein biomarkers for the three MASLD molecular subtypes was shown in FIG. 15. It could be seen that the optimal combination of the serum protein biomarkers for the three MASLD molecular subtypes was CPM, NUCB1, KRT17 and CLC (Train AUC = 0.904, and Test AUC = 0.806). Compared with the other two MASLD molecular subtypes, the levels of CPM, NUCB1, KRT17 and CLC in the serum of patients in the MASLD-mSII group were the highest (FIG. 16).

### 2.2 Combination of serum lipid biomarkers

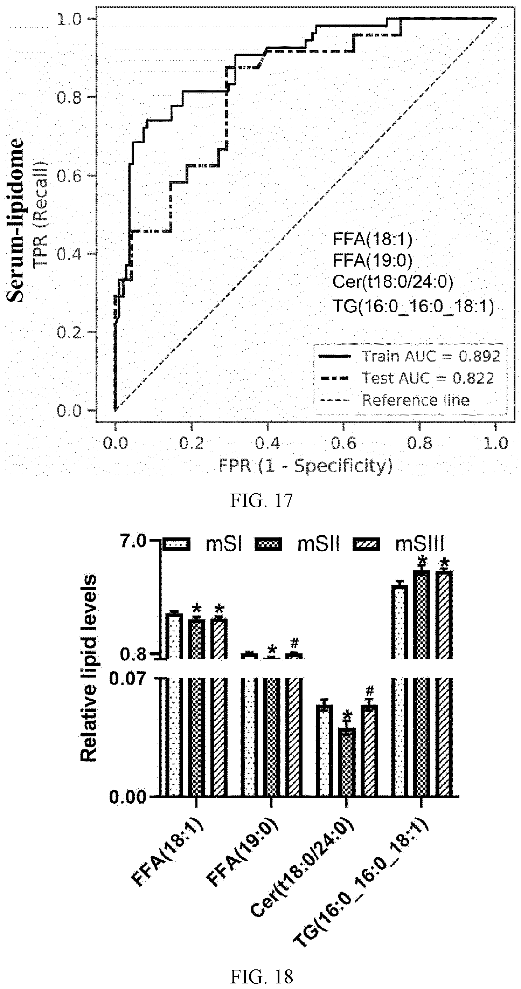

An identification result of an optimal combination of serum lipid biomarkers for the three MASLD molecular subtypes was shown in FIG. 17. It could be seen that the optimal combination of the serum lipid biomarkers for the three MASLD molecular subtypes was FFA (18:1), FFA (19:0), Cer (t18:0/24:0) and TG (16:0_16:0_18:1) (Train AUC = 0.892, and Test AUC = 0.822). Compared with the other two groups, FFA (18:1) or TG (16:0_16:0_18:1) had the highest or lowest levels in the serum of patients in the MASLD-mSI group; and FFA (19:0) and Cer (t18:0/24:0) had the lowest levels in the serum of patients in the MASLD-mSII group.

### 2.3 Combination of serum metabolite biomarkers

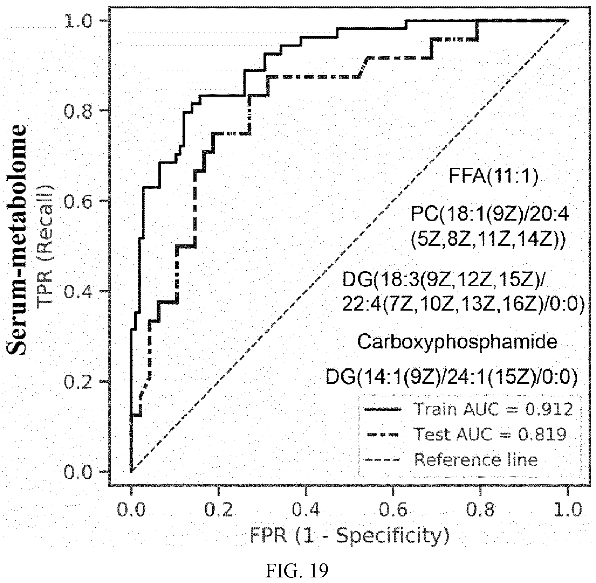

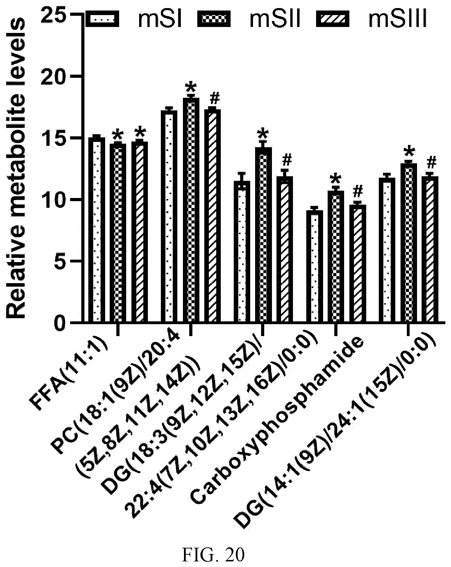

An identification result of an optimal combination of serum metabolite biomarkers for the three MASLD molecular subtypes was shown in FIG. 19. It could be seen that the optimal combination of the serum metabolite biomarker for the three MASLD molecular subtypes was FFA (11:1), PC (18:1 (9Z)/20:4 (5Z, 8Z, 11Z, 14Z)), DG (18:3 (9Z, 12Z, 15Z)/22:4 (7Z, 10Z, 13Z, 16Z)/0:0), Carboxyphosphamide and DG (14:1 (9Z)/24:1 (15Z)/0:0 ) (Train AUC = 0.912, and Test AUC = 0.819). Compared with the other two MASLD molecular subtypes, the level of FFA (11:1) was the highest in the serum of patients in the MASLD-mSI group; and PC (18:1 (9)/20:4 (5Z, 8Z, 11Z, 14Z)), DG (18:3 (9Z, 12Z, 15Z)/22:4 (7Z, 10Z, 13Z, 16Z)/0:0), Carboxyphosphamide and DG (14:1 (9Z)/24:1 (15Z)/0:0) had the highest levels in the serum of patients in the MASLD-mSII group (FIG. 20).

### 2.4 Combination of serum multiomics (including protein, lipid and metabolite) biomarkers

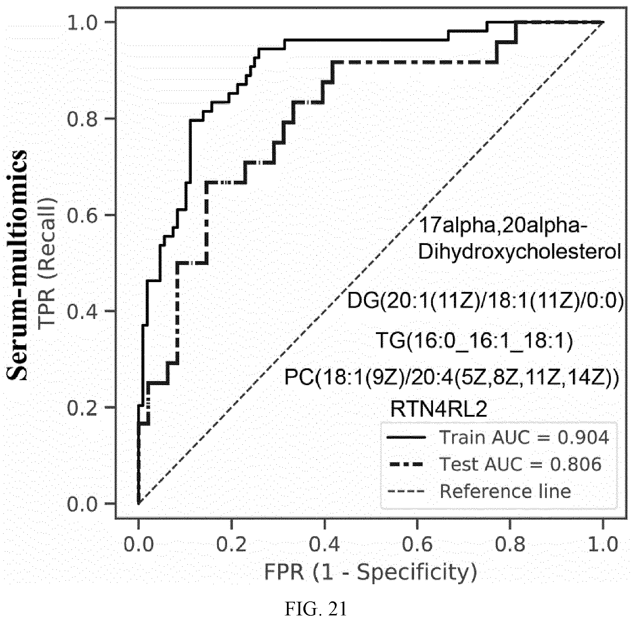

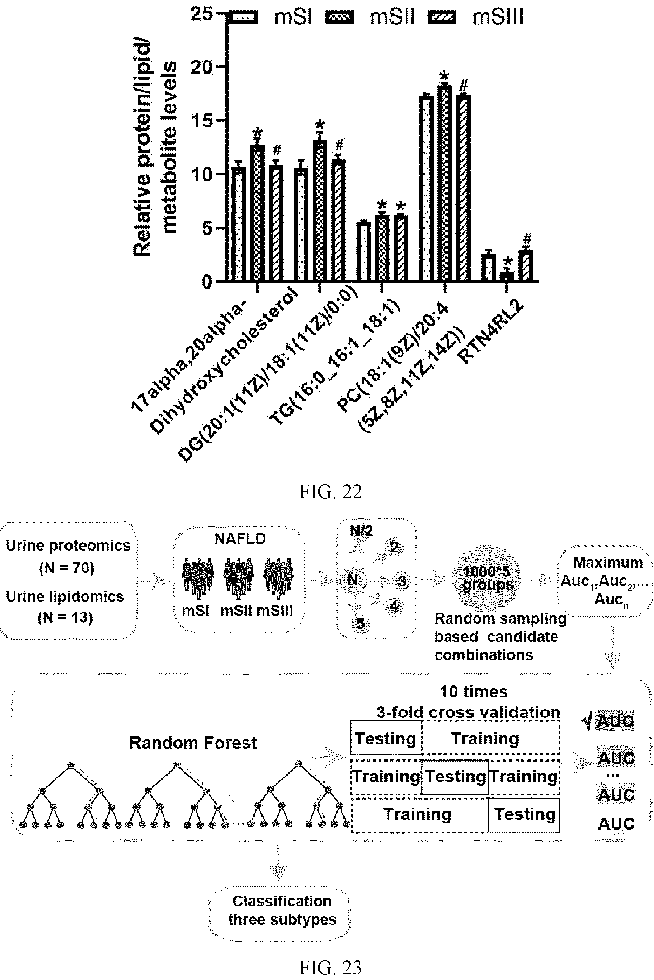

An identification result of an optimal combination of serum multiomics (including protein, lipid and metabolite) biomarkers for the three MASLD molecular subtypes was shown in FIG. 21. It could be seen that the optimal combination of the serum multiomics (including protein, lipid and metabolite) biomarkersfor the three MASLD molecular subtypes was 17alpha, 20alpha-Dihydroxycholesterol, DG (20:1 (11Z)/18:1 (11Z)/0:0), TG(16:0_16:1_18:1), PC (18:1 (9Z)/20:4 (5Z, 8Z, 11Z, 14Z)) and RTN4RL2 combination (Train AUC = 0.904, and Test AUC = 0.806). Compared with the other two MASLD molecular subtypes, the levels of 17alpha, 20alpha-Dihydroxycholesterol, DG (20:1 (11Z)/18:1 (11Z)/0:0), PC (18:1 (9Z)/20:4 (5Z, 8Z, 11Z, 14Z)) and RTN4RL2 were the highest in the serum of patients in the MASLD-mSII group; and the level of TG (16: 0_16:1_18:1) in the serum of patients in the MASLD-mSI group was the lowest (FIG. 22).

### 3. Combination of urine protein biomarkers, urine metabolite biomarkers and urine multiomics (including protein and metabolite) biomarkers

Proteomics analysis was performed on urine of 46 patients with MASLD, and omics analysis was performed on urine of 48 patients with MASLD on lipid and metabolism. Compared with the other two MASLD molecular subtypes, characteristic urine proteins, urine lipids, and urine metabolites were all defined as differential molecules.. Since the three MASLD molecular subtypes had no characteristic lipids, there were no candidate lipid biomarkers. The characteristic urine protein, urine metabolite and urine multiomics (including protein and metabolite) for the three MASLD molecular subtypes were used as candidate biomarkers, and Random Forest analysis was used. Model parameters: the RandomForestClassifier function in python language was used, where n estimators = 20, max depth = 2, min samples leaf = 2, and the rest used default values. The output result was the probability that the patient belonged to three subtypes of MASLD, and the subtype with the highest probability was the subtype of the patient. An optimal biomarker combination including a combination of urine proteome biomarkers, a combination of urine metabolome biomarkers, and a combination of urine multiomics (including protein and metabolite) biomarkers for the three MASLD molecular subtypes was clearly identified. An identification flow chart was shown in FIG. 23.

### 3.1 Combination of urine protein biomarkers

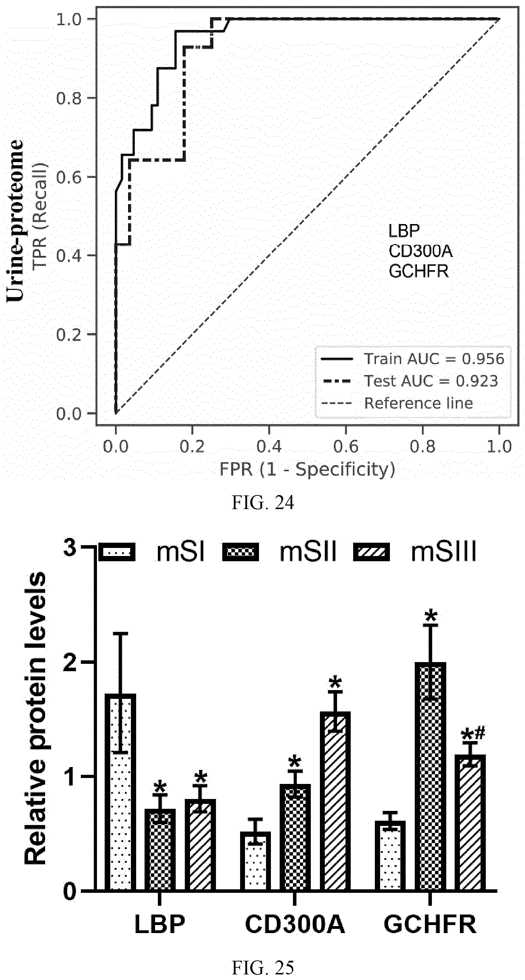

An identification result of an optimal combination of urine protein biomarkers for the three MASLD molecular subtypes was shown in FIG. 24. It could be seen that the optimal combination of the urine protein biomarkers for the three MASLD molecular subtypes was the combination of LBP, CD300A and GCHFR (Train AUC = 0.956, and Test AUC = 0.923). Compared with the other two MASLD molecular subtypes, the level of LBP in urine of patients in the MASLD-mSI group was the highest, the level of GCHFR in urine of patients in the MASLD-mSII group was the highest, and the level of CD300A in urine of patients in the MASLD-mSIII group was the highest (FIG. 25).

### 3.2 Combination of urine metabolite biomarkers

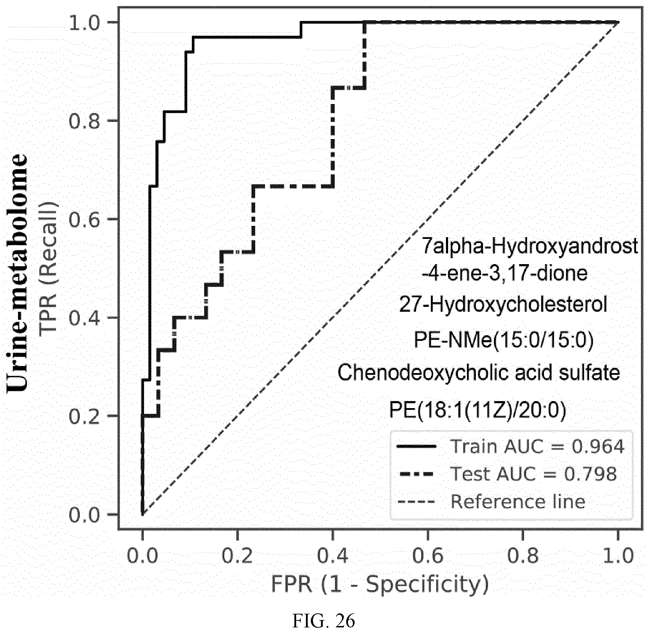

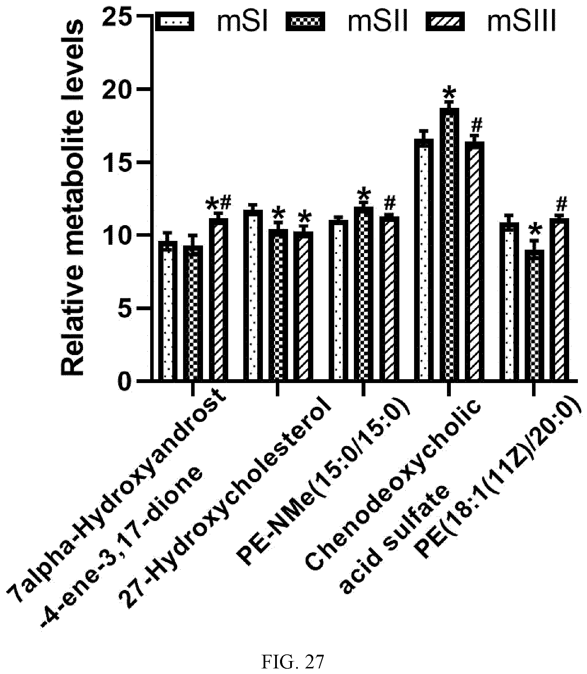

An identification result of an optimal combination of urine metabolite biomarkers for the three MASLD molecular subtypes was shown in FIG. 26. It could be seen that the optimal combination of the urine metabolite biomarkers for the three MASLD molecular subtypes was 7alpha-Hydroxyandrost-4-ene-3,17-dione, 27-Hydroxycholesterol, PE-NMe (15:0/15:0), Chenodeoxycholic acid sulfate and PE (18:1 (11Z)/20:0) combination (Train AUC = 0.964, and Test AUC = 0.798). Compared with the other two MASLD molecular subtypes, the level of 27-Hydroxycholesterol in the urine of patients in the MASLD-mSI group was the highest, the levels of PE-NMe (15:0/15:0) and Chenodeoxycholic acid sulfate in the urine of patients in the MASLD-mSII group were the highest, and the level of PE (18:1 (11Z)/20:0) in the urine of patients in the MASLD-mSII group was the lowest, and the level of 7alpha-Hydroxyandrost-4-ene-3, 17-dione in the urine of patients in the MASLD-mSIII group was the highest (FIG. 27).

### 3.3 Combination of urine multiomics (including protein and metabolite) biomarkers

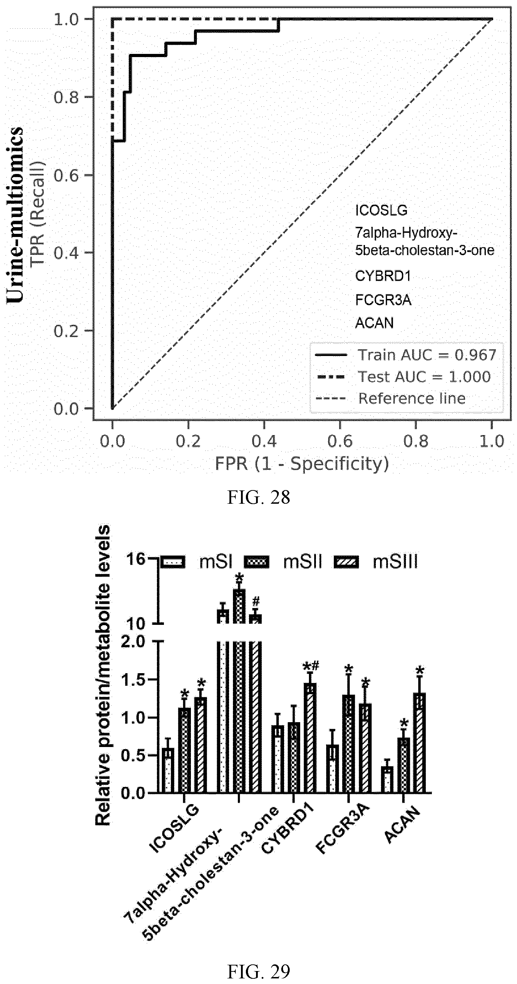

An identification result of an optimal combination of urine multiomics (including protein and metabolite) biomarkers for the three MASLD molecular subtypes was shown in FIG. 28. It could be seen that the optimal combination of urine multiomics (including protein and metabolite) biomarkers for the three MASLD molecular subtypes was ICOSLG, 7alpha-Hydroxy-5beta-cholstan-3-one, CYBRD1, FCGR3A and ACAN (Train AUC = 0.967, and Test AUC = 1.000). Compared with the other two MASLD molecular subtypes, the levels of ICOSLG, FCGR3 A and ACAN in the urine of patients in the MASLD-mSI group were the lowest, the level of 7alpha-Hydroxy-5beta-cholstan-3-one in the urine of patients in the MASLD-mSII group was the highest, and the level of CYBRD1 in the urine of patients in the MASLD-mSIII group was the highest (FIG. 29).

It could be seen from the above that the present disclosure gave biomarkers for the diagnosis of three molecular subtypes of MASLD from multiple levels (liver protein, serum and urine protein, lipid, and metabolite). The accuracy of the combination of the liver protein biomarkers LAMB1, FLNC and ERCC3 was 92.98%, and the AUC was 0.991; the Train AUC of the combination of the serum protein biomarkers CPM, NUCB1, KRT17 and CLC was 0.904, and Test AUC was 0.806; the Train AUC of the combination of the serum lipid biomarkers free fatty acid/FFA (18:1), free fatty acid/FFA (19:0), ceramide/Cer (t18:0/24:0) and triglyceride/TG (16:0_16:0_18:1) was 0.892, and the Test AUC was 0.822; the Train AUC of the combination of the serum metabolite biomarkers free fatty acid/FFA (11:1), phosphatidylcholine/PC (18:1 (9Z)/20:4 (5Z, 8Z, 11Z, 14Z)), diacylglycerol/DG (18:3 (9Z, 12Z, 15Z)/22:4 (7Z, 10Z, 13Z, 16Z)/0:0), carboxyphosphamide/Carboxyphosphamide, diacylglycerol/DG (14:1 (9Z)/24:1 (15Z)/0: 0) was 0.912, and the Test AUC was 0.819; the AUC of the combination of the serum multiomics (including protein, lipid and metabolite) biomarkers 17α, 20α-dihydroxycholesterol/17alpha, 20alpha-dihydroxycholesterol, diglyceride/DG (20:1 (11Z)/18:1 (11Z)/0:0), triglyceride/TG (16:0_16:1_18:1), phosphatidylcholine/PC (18:1 (9Z)/20:4 (5Z, 8Z, 11Z, 14Z)) and RTN4RL2 was 0.904, and the Test AUC was 0.806; the Train AUC of the combination of the urine protein biomarkers CPM, NUCB1, KRT17 and CLC was 0.956, and the Test AUC was 0.923; the Train AUC of the combination of the urine metabolite biomarkers 7α-Hydroxyandrost-4-ene-3, 17-dione, 27-Hydroxycholesterol, PE-NMe (15:0/15:0), Chenodeoxycholic acid sulfate, PE (18:1 (11Z)/20:0) was 0.964, and the Test AUC was 0.798; and the Train AUC of the combination of the urine multiomics (including protein and metabolite) biomarkers ICOSLG, 7alpha-hydroxy-5beta-cholstan-3-one, CYBRD1, FCGR3 A and ACAN was 0.967, and the Test AUC was 1.000. The above combinations of biomarkers had good diagnostic effects on the three molecular subtypes of MASLD.

### Embodiment 3

Liver paraffin sections of MASLD patients (92 cases) from three other hospitals were collected. Multiple immunofluorescence staining of LAMB 1, FLNC and ERCC3 was performed on liver sections, and quantitative analysis was performed (LAMB1 was used to calculate the positive staining area, FLNC was used to calculate the average optical density, and ERCC3 was used to calculate the percentage of nuclear positive staining). Subsequently, the quantitative values of multiple immunofluorescence staining of LAMB1, FLNC and ERCC3 were analyzed by Random Forest to identify the molecular subtypes of each MASLD patient. The results showed that MASLD-mSI n = 25, MASLD-mSII n = 19, MASLD-mSIII n = 48 (Table 1). The levels and clinical phenotypes of LAMB 1, FLNC and ERCC3 in the three molecular subtypes of MASLD in the external cohort were consistent with those in the internal cohort.

**Table 1 Identification results of molecular subtypes of 92 patients with MASLD**

| Patient number | Pre_type | mSI_proba | mSII_proba | mSIII_proba |
|---|---|---|---|---|
| 1 | mSI | 0.849069 | 0.013636 | 0.137295 |
| 2 | mSI | 0.764055 | 0.068354 | 0.167592 |
| 3 | mSI | 0.842947 | 0.01447 | 0.142584 |
| 4 | mSI | 0.691439 | 0.068527 | 0.240034 |
| 5 | mSI | 0.582544 | 0.09291 | 0.324546 |
| 6 | mSI | 0.794491 | 0.013724 | 0.191785 |
| 7 | mSI | 0.703741 | 0.017169 | 0.27909 |
| 8 | mSI | 0.816179 | 0.008974 | 0.174847 |
| 9 | mSI | 0.79039 | 0.02177 | 0.18784 |
| 10 | mSI | 0.626091 | 0.042685 | 0.331223 |
| 11 | mSI | 0.693969 | 0.012034 | 0.293996 |
| 12 | mSI | 0.667055 | 0.031884 | 0.301061 |
| 13 | mSI | 0.813743 | 0.009044 | 0.177213 |
| 14 | mSI | 0.818939 | 0.012803 | 0.168258 |
| 15 | mSI | 0.755146 | 0.067227 | 0.177627 |
| 16 | mSI | 0.800559 | 0.019373 | 0.180068 |
| 17 | mSI | 0.783521 | 0.022988 | 0.193491 |
| 18 | mSI | 0.825757 | 0.012803 | 0.16144 |
| 19 | mSI | 0.49885 | 0.135382 | 0.365768 |
| 20 | mSI | 0.801972 | 0.023437 | 0.174591 |
| 21 | mSI | 0.8558 | 0.012459 | 0.131741 |
| 22 | mSI | 0.79659 | 0.017553 | 0.185857 |
| 23 | mSI | 0.866632 | 0.015455 | 0.117914 |
| 24 | mSI | 0.734495 | 0.009873 | 0.255632 |
| 25 | mSI | 0.80144 | 0.022988 | 0.175571 |
| 26 | mSII | 0.165009 | 0.642599 | 0.192392 |
| 27 | mSII | 0.099622 | 0.59483 | 0.305549 |
| 28 | mSII | 0.044825 | 0.705725 | 0.24945 |
| 29 | mSII | 0.046297 | 0.650554 | 0.303149 |
| 30 | mSII | 0.112192 | 0.584318 | 0.30349 |
| 31 | mSII | 0.035506 | 0.673427 | 0.291067 |
| 32 | mSII | 0.035506 | 0.717496 | 0.246997 |
| 33 | mSII | 0.045242 | 0.700309 | 0.25445 |
| 34 | mSII | 0.113386 | 0.62633 | 0.260284 |
| 35 | mSII | 0.08339 | 0.751929 | 0.164681 |
| 36 | mSII | 0.060946 | 0.789268 | 0.149786 |
| 37 | mSII | 0.086189 | 0.742869 | 0.170942 |
| 38 | mSII | 0.041062 | 0.712959 | 0.245979 |
| 39 | mSII | 0.052758 | 0.70177 | 0.245473 |
| 40 | mSII | 0.023693 | 0.812187 | 0.164119 |
| 41 | mSII | 0.086189 | 0.742869 | 0.170942 |
| 42 | mSII | 0.028276 | 0.684905 | 0.286819 |
| 43 | mSII | 0.046816 | 0.763637 | 0.189547 |
| 44 | mSII | 0.039351 | 0.803629 | 0.15702 |
| 45 | mSIII | 0.409604 | 0.084953 | 0.505442 |
| 46 | mSIII | 0.366353 | 0.00837 | 0.625277 |
| 47 | mSIII | 0.347123 | 0.029793 | 0.623084 |
| 48 | mSIII | 0.34855 | 0.010204 | 0.641246 |
| 49 | mSIII | 0.416777 | 0.002186 | 0.581037 |
| 50 | mSIII | 0.414864 | 0.011227 | 0.573908 |
| 51 | mSIII | 0.40791 | 0.01087 | 0.58122 |
| 52 | mSIII | 0.450445 | 0.002928 | 0.546627 |
| 53 | mSIII | 0.418508 | 0.01087 | 0.570622 |
| 54 | mSIII | 0.363948 | 0.007537 | 0.628515 |
| 55 | mSIII | 0.198817 | 0.057843 | 0.74334 |
| 56 | mSIII | 0.359234 | 0.007537 | 0.633229 |
| 57 | mSIII | 0.122497 | 0.335454 | 0.542049 |
| 58 | mSIII | 0.382132 | 0.007537 | 0.610331 |
| 59 | mSIII | 0.355277 | 0.007537 | 0.637186 |
| 60 | mSIII | 0.212939 | 0.045765 | 0.741296 |
| 61 | mSIII | 0.304486 | 0.018932 | 0.676582 |
| 62 | mSIII | 0.332444 | 0.033365 | 0.634191 |
| 63 | mSIII | 0.383909 | 0.032414 | 0.583677 |
| 64 | mSIII | 0.0848 | 0.406688 | 0.508512 |
| 65 | mSIII | 0.475737 | 0.047206 | 0.477057 |
| 66 | mSIII | 0.345873 | 0.029793 | 0.624334 |
| 67 | mSIII | 0.394156 | 0.00587 | 0.599974 |
| 68 | mSIII | 0.393735 | 0.008279 | 0.597987 |
| 69 | mSIII | 0.455541 | 0.011827 | 0.532632 |
| 70 | mSIII | 0.356662 | 0.007537 | 0.635802 |
| 71 | mSIII | 0.479951 | 0.02216 | 0.497889 |
| 72 | mSIII | 0.292257 | 0.027265 | 0.680478 |
| 73 | mSIII | 0.451228 | 0.003382 | 0.54539 |
| 74 | mSIII | 0.402459 | 0.002186 | 0.595355 |
| 75 | mSIII | 0.213059 | 0.081787 | 0.705153 |
| 76 | mSIII | 0.34855 | 0.010204 | 0.641246 |
| 77 | mSIII | 0.213059 | 0.081787 | 0.705153 |
| 78 | mSIII | 0.179431 | 0.152075 | 0.668495 |
| 79 | mSIII | 0.247581 | 0.184052 | 0.568367 |
| 80 | mSIII | 0.220041 | 0.013847 | 0.766111 |
| 81 | mSIII | 0.436586 | 0.094094 | 0.46932 |
| 82 | mSIII | 0.246138 | 0.027636 | 0.726226 |
| 83 | mSIII | 0.093065 | 0.031691 | 0.875244 |
| 84 | mSIII | 0.38524 | 0.021136 | 0.593625 |
| 85 | mSIII | 0.406839 | 0.011227 | 0.581934 |
| 86 | mSIII | 0.360569 | 0.010561 | 0.628871 |
| 87 | mSIII | 0.191498 | 0.025136 | 0.783366 |
| 88 | mSIII | 0.078321 | 0.034578 | 0.887101 |
| 89 | mSIII | 0.15012 | 0.033507 | 0.816373 |
| 90 | mSIII | 0.462186 | 0.015855 | 0.521959 |
| 91 | mSIII | 0.03569 | 0.043148 | 0.921162 |
| 92 | mSIII | 0.248942 | 0.077826 | 0.673232 |

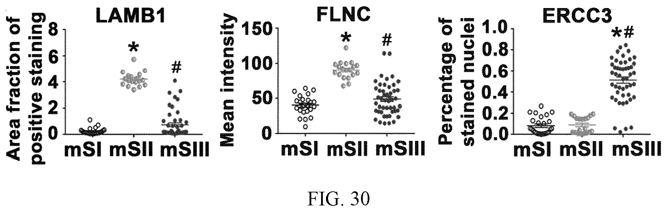

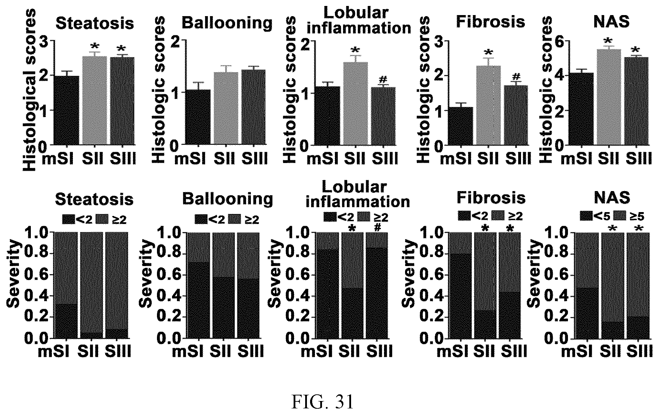

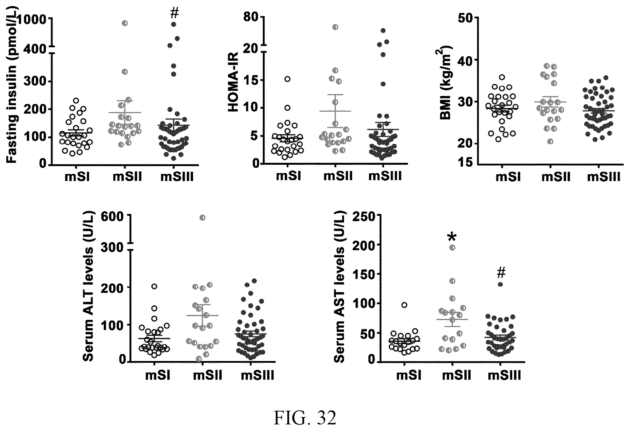

The expression levels, histologic characteristics, and serum biochemical characteristics of liver proteins LAMB1, FLNC and ERCC3 of patients with three MASLD molecular subtypes were measured. The determined results were shown in FIG. 30-FIG. 32. It could be seen from FIG. 30 that the levels of LAMB1 and FLNC in the MASLD-mSII group were significantly higher than those in the MASLD-mSI and MASLD-mSIII groups, and the level of ERCC3 in the MASLD-mSIII group was significantly higher than that in the MASLD-mSI and MASLD-mSII groups. It could be seen from FIG. 31 that the steatosis and NAS scores of the MASLD-mSI group were lower than those of the MASLD-mSII and MASLD-mSIII groups, while there was no significant difference between the MASLD-mSII and MASLD-mSIII groups. In addition, the scores of liver fibrosis and lobular inflammation in the MASLD-mSII group were higher than those in the MASLD-mSI and MASLD-mSIII groups, while there was no significant change between the MASLD-mSI and MASLD-mSIII groups. The proportion of patients with severe liver fibrosis and inflammation in the MASLD-mSII group was significantly higher than that in the MASLD-mSI and MASLD-mSIII groups. There was no significant difference in the score results for liver ballooning degeneration among the three MASLD molecular subtypes. It could be seen from FIG. 32 that the levels of fasting insulin, HOMA-IR, ALT and AST in the MASLD-mSI group tended to be lower than those in the MASLD-mSII and MASLD-mSIII groups. The BMI value of the MASLD-mSII group tended to be higher than that of the MASLD-mSI and MASLD-mSIII groups.

It can be seen that the combination of liver protein LAMB 1, FLNC and ERCC3 biomarkers can effectively distinguish the three MASLD molecular subtypes.

The above descriptions are only the preferred embodiments of the present disclosure. It is to be pointed out that those of ordinary skill in the art can also make several improvements and modifications without departing from the principle of the present disclosure, and such improvements and modifications shall fall within the protection scope of the present disclosure.

## Claims

1. An application of a biomarker in preparing metabolic dysfunction-associated steatotic liver disease classification products, and the biomarker is any one or more of the following: a combination of liver protein biomarkers, a combination of serum protein biomarkers, a combination of serum lipid biomarkers, a combination of serum metabolite biomarkers, a combination of serum multiomics (including protein, lipid and metabolite) biomarkers, a combination of urine protein biomarkers, a combination of urine metabolite biomarkers, and a combination of urine multiomics (including protein and metabolite) biomarkers; and
a classification of a metabolic dysfunction-associated steatotic liver disease is divided into metabolically active type, a high-risk type of cirrhosis, and high-risk type of hepatocellular carcinoma.

2. The application according to claim 1, wherein the products comprise a kit;
the combination of liver protein biomarkers is a combination of laminin beta 1 (LAMB1), filamin C (FLNC), and excision repair cross-complementation group 3 (ERCC3);
the combination of serum protein biomarkers is a combination of carboxypeptidase M (CPM), nucleobindin 1 (NUCB1), keratin 17 (KRT17), and Galectin-10 (CLC);
the combination of serum lipid biomarkers is a combination of free fatty acid (FFA) (18:1), FFA(19:0), ceramide (Cer) (t18:0/24:0) and triacylglycerol (TG) (16:0_16:0_18:1);
the combination of serum metabolite biomarkers is a combination of FFA (11:1), phosphatidylcholine (PC) (18:1 (9Z)/20:4 (5Z, 8Z, 11Z, 14Z)), diglycerides (DG) (18:3 (9Z,12Z,15Z)/22:4 (7Z, 10Z, 13Z, 16Z)/0:0), Carboxyphosphamide, and DG (14: 1(9Z)/24:1 (15Z) /0:0);
the combination of serum multiomics (including protein, lipid and metabolite) biomarkers is a combination of 17alpha, 20alpha-Dihydroxycholesterol, DG (20:1 (11Z)/18:1 (11Z)/0:0), TG (16:0_16:1_18:1), PC (18:1 (9Z)/20:4 (5Z,8Z,11Z,14Z)) and reticulon 4 receptor-like 2 (RTN4RL2);
the combination of urine protein biomarkers is a combination of lipopolysaccharide binding protein (LBP), CD300 antigen-like family member A (CD300A), and GTP cyclohydrolase 1 feedback regulator (GCHFR);
the combination of urine metabolite biomarkers is a combination of 7alpha-Hydroxyandrost-4-ene-3, 17-dione, 27-Hydroxycholesterol, monomethyl phosphatidylethanolamine (PE-NMe) (15:0/15:0), Chenodeoxycholic acid sulfate and Phosphatidylethanolamine (PE) (18:1 (11Z)/20:0); and
the combination of urine multiomics (including protein and metabolite) biomarkers is a combination of ICOS ligand (ICOSLG), 7alpha-Hydroxy-5beta-cholstan-3-one, cytochrome b reductase 1 (CYBRD1), Fc gamma receptor III-A (FCGR3A), and aggrecan (ACAN).

3. A combination of liver protein biomarkers for the metabolic dysfunction-associated steatotic liver disease classification, and the combination of liver protein biomarkers is a combination of LAMB 1, FLNC and ERCC3.

4. A combination of serum protein biomarkers for the metabolic dysfunction-associated steatotic liver disease classification, and the combination of serum protein biomarkers is a combination of CPM, NUCB1, KRT17 and CLC.

5. A combination of serum lipid biomarkers for the metabolic dysfunction-associated steatotic liver disease classification, and the combination of serum lipid biomarkers is a combination of FFA (18:1), FFA (19:0), Cer (118:0/24:0) and TG (16:0_16:0_18:1).

6. A combination of serum metabolite biomarkers for the metabolic dysfunction-associated steatotic liver disease classification, and the combination of serum metabolite biomarkers is a combination of FFA (11:1), PC (18:1 (9Z)/20:4 (5Z, 8Z, 11Z, 14Z)), DG (18:3 (9Z,12Z,15Z)/22:4 (7Z, 10Z, 13Z, 16Z)/0:0), Carboxyphosphamide and DG (14: 1(9Z)/24:1 (15Z)/0:0).

7. A combination of serum multiomics (including protein, lipid and metabolite) biomarkers for the metabolic dysfunction-associated steatotic liver disease classification, and the combination of serum multiomics (including protein, lipid and metabolite) biomarkers is a combination of 17alpha, 20alpha-Dihydroxycholesterol, DG (20:1 (11Z)/18:1 (11Z)/0:0), TG (16:0_16:1_18:1), PC (18:1 (9Z)/20:4 (5Z,8Z,11Z,14Z)) and RTN4RL2.

8. A combination of urine protein biomarkers for the metabolic dysfunction-associated steatotic liver disease classification, and the combination of urine protein biomarkers is a combination of LBP, CD300A and GCHF.

9. A combination of urine metabolite biomarkers for the metabolic dysfunction-associated steatotic liver disease classification, and the combination of urine metabolite biomarkers is a combination of 7alpha-Hydroxyandrost-4-ene-3,17-dione, 27-Hydroxycholesterol, PE-NMe (15:0/15:0), Chenodeoxycholic acid sulfate, and PE (18:1 (11Z)/20:0).

10. A combination of urine multiomics (including protein and metabolite) biomarkers for the metabolic dysfunction-associated steatotic liver disease classification, and the combination of urine multiomics (including protein and metabolite) biomarkers is a combination of ICOSLG, 7alpha-Hydroxy-5beta-cholstan-3-one, CYBRD1, FCGR3A and ACAN.

## Amended claims

### Amended claims in accordance with Rule 137(2) EPC.

1. A method for classifying metabolic dysfunction-associated steatotic liver disease into metabolically active type, a high-risk type of cirrhosis, and high-risk type of hepatocellular carcinoma using as biomarker any one or more of the following: a combination of liver protein biomarkers, a combination of serum protein biomarkers, a combination of serum lipid biomarkers, a combination of serum metabolite biomarkers, a combination of serum multiomics (including protein, lipid and metabolite) biomarkers, a combination of urine protein biomarkers, a combination of urine metabolite biomarkers, and a combination of urine multiomics (including protein and metabolite) biomarkers provided in a kit
**characterized in that**
the combination of liver protein biomarkers is a combination of laminin beta 1 (LAMB1), filamin C (FLNC), and excision repair cross-complementation group 3 (ERCC3);
the combination of serum protein biomarkers is a combination of carboxypeptidase M (CPM), nucleobindin 1 (NUCB1), keratin 17 (KRT17), and Galectin-10 (CLC);
the combination of serum lipid biomarkers is a combination of free fatty acid (FFA) (18:1), FFA (19:0), ceramide (Cer) (t18:0/24:0) and triacylglycerol (TG) (16:0_16:0_18:1);
the combination of serum metabolite biomarkers is a combination of FFA (11:1), phosphatidylcholine (PC) (18:1 (9Z)/20:4 (5Z, 8Z, 11Z, 14Z)), diglycerides (DG) (18:3 (9Z,12Z,15Z)/22:4 (7Z, 10Z, 13Z, 16Z)/0:0), Carboxyphosphamide, and DG (14: 1(9Z)/24:1 (15Z) /0:0);
the combination of serum multiomics (including protein, lipid and metabolite) biomarkers is a combination of 17alpha, 20alpha-Dihydroxycholesterol, DG (20:1 (11Z)/18:1 (11Z)/0:0), TG (16:0_16:1_18:1), PC (18:1 (9Z)/20:4 (5Z,8Z,11Z,14Z)) and reticulon 4 receptor-like 2 (RTN4RL2);
the combination of urine protein biomarkers is a combination of lipopolysaccharide binding protein (LBP), CD300 antigen-like family member A (CD300A), and GTP cyclohydrolase 1 feedback regulator (GCHFR);
the combination of urine metabolite biomarkers is a combination of 7alpha-Hydroxyandrost-4-ene-3, 17-dione, 27-Hydroxycholesterol, monomethyl phosphatidylethanolamine (PE-NMe) (15:0/15:0), Chenodeoxycholic acid sulfate and Phosphatidylethanolamine (PE) (18:1 (11Z)/20:0); and
the combination of urine multiomics (including protein and metabolite) biomarkers is a combination of ICOS ligand (ICOSLG), 7alpha-Hydroxy-5beta-cholstan-3-one, cytochrome b reductase 1 (CYBRD1), Fc gamma receptor III-A (FCGR3A), and aggrecan (ACAN).

2. A combination of isolated biomarkers for use in a method of classifying the metabolic dysfunction-associated steatotic liver disease according to claim 1,
wherein
the combination of liver protein biomarkers is a combination of LAMB1, FLNC and ERCC3;
the combination of serum protein biomarkers is a combination of CPM, NUCB1, KRT17 and CLC;
the combination of serum lipid biomarkers is a combination of FFA (18:1), FFA (19:0), Cer (t18:0/24:0) and TG (16:0_16:0_18:1);
the combination of serum metabolite biomarkers is a combination of FFA (11:1), PC (18:1 (9Z)/20:4 (5Z, 8Z, 11Z, 14Z)), DG (18:3 (9Z,12Z,15Z)/22:4 (7Z, 10Z, 13Z, 16Z)/0:0), Carboxyphosphamide and DG (14: 1(9Z)/24:1 (15Z)/0:0;
the combination of serum multiomics (including protein, lipid and metabolite) biomarkers is a combination of 17alpha, 20alpha-Dihydroxycholesterol, DG (20:1 (11Z)/18:1 (11Z)/0:0), TG (16:0_16:1_18:1), PC (18:1 (9Z)/20:4 (5Z,8Z,11Z,14Z)) and RTN4RL2;
the combination of urine protein biomarkers is a combination of LBP, CD300A and GCHF; the combination of urine metabolite biomarkers is a combination of 7alpha-Hydroxyandrost-4-ene-3,17-dione, 27-Hydroxycholesterol, PE-NMe (15:0/15:0), Chenodeoxycholic acid sulfate, and PE (18:1 (11Z)/20:0); and
the combination of urine multiomics (including protein and metabolite) biomarkers is a combination of ICOSLG, 7alpha-Hydroxy-5beta-cholstan-3-one, CYBRD1, FCGR3A and ACAN.
